## Europäisches Patentamt
## European Patent Office
## Office européen des brevets

(19)

(11) Numéro de publication: **0 014 124**
**B1**

(12)
# FASCICULE DE BREVET EUROPEEN

(45) Date de publication du fascicule du brevet: **09.02.83**

(51) Int. Cl.³: **A 63 B 71/12,**
**A 41 D 17/00, A 63 C 11/00**

(21) Numéro de dépôt: **80400053.7**

(22) Date de dépôt: **15.01.80**

(54) Jambière pour la pratique du ski.

(30) Priorité: **17.01.79 FR 7901073**

(43) Date de publication de la demande:
**06.08.80 Bulletin 80/16**

(45) Mention de la délivrance du brevet:
**09.02.83 Bulletin 83/6**

(84) Etats contractants désignés:
**AT CH DE IT SE**

(56) Documents cités:
**DE - A - 1 900 074**
**DE - A - 1 935 278**
**FR - A - 820 979**
**FR - A - 1 549 497**
**US - A - 1 931 524**
**US - A - 2 902 779**

(73) Titulaire: **Pano, Pierre**
**46 Boulevard Excelmans**
**F-75016 Paris (FR)**

(72) Inventeur: **Pano, Pierre**
**46 Boulevard Excelmans**
**F-75016 Paris (FR)**

(74) Mandataire: **Lemonnier, André**
**4 Boulevard Saint Denis**
**F-75010 Paris (FR)**

Courier Press, Leamington Spa, England.

Jambière pour la pratique du ski

Au cours des années récentes, on a mis au point et développé des chaussures de ski de plus en plus complexes pour remédier aux accidents tels que les entorses de la cheville. Ces chaussures ont été réalisées en matière rigide notamment en plastique et comportent une manchette enserrant la partie inférieure de la jambe, articulée sur la coquille de pied autour de l'axe d'articulation des malléoles. Cette manchette a une hauteur limitée en raison de la complexité du mouvement d'articulation du tibia et du péroné autour de l'axe des malléoles et il est nécessaire de prévoir des rembourrages souples pour éviter de blesser la jambe et autoriser un certain jeu. Ces chaussures se sont effectivement avérées très efficaces et on ne constate pratiquement plus d'accident au niveau de la cheville. Par contre, lors d'une chute au cours de laquelle les sécurités ne jouent pas, la totalité due couple du poids du corps s'exerce au niveau de la section supérieure de la manchette, en un endroit où les sections du tibia et du péroné sont relativement faibles, et on constate un nombre croissant de cassures ou de fêlures de ces os à ce niveau précis. Un risque de luxation du genou existe également dan le cas d'une chute avant ou latérale. Enfin un troisième type d'accident résulte d'un effort de torsion au niveau de la cheville, les sécurités conçues pour fonctionner sous l'effet d'un couple dans le plan longitudinal par soulèvement du talon et éventuellement de la pointe ne présentant pas assez de sensibilité pour un réglage desdites sécurités qui ne soit pas trop lâche pour éviter des déchaussages fréquents dans la pratique normale de ce sport.

D'autre part, la demande de brevet allemand N° 1.935.278 décrit une jambière qui peut être utilisée aussi bien pour le football, le hockey ou le ski, jambière qui, dans le cas du ski, comporte une coquille en un matériau élastique dur, munie de flasques latéraux descendant dans le haut de la tige de la chaussure de ski, en dessous du niveau des malléoles. En fait, cette jambière a été conçue pour remédier aux inconvénients des chaussures de ski anciennes et elle présente les mêmes avantages que la manchette enserrant la partie inférieure de la jambe des chaussures de ski en matière rigide actuelle. Toutefois, avec ce type de jambière, on n'évite pas les fractures de la jambe à un niveau supérieur à celui de la cheville, du fait de la déformation élastique de la jambière lors d'une chute avec application d'un couple de torsion au niveau de la cheville.

La présente invention a pour but d'éviter ce genre d'accidents et elle a pour objet une coquille formant jambière, descendant dans le haut de la tige de la chaussure de ski, en dessous du niveau des malléoles, en s'emboîtant dans la chaussure par deux flasques latéraux prenant appui contre les flancs de la tige, la coquille étant constituée par deux demi-coquilles avant et arrière en un matériau rigide assemblées entre elles de façon rigide de manière à enserrer la jambe sur tout son pourtour, lesdits flasques latéraux formant partie intégrante de la demi-coquille arrière et la demi-coquille avant étant solidaire d'une partie supérieure avant emboîtant le genou en avant et latéralement. Cette jambière relève la section où s'exerce le couple du poids du corps jusqu'au droit de la section supérieure de la jambière, ce qui réduit dans une très forte proportion le valeur de la composante de ce couple dont le centre d'application peut être considéré comme confondu avec les malléoles quel que soit le type de la chute, avant, arrière ou latérale. Dans le cas d'une torsion, la jambière présentant un angle de rotation limité par rapport à la tige de la chaussure, le couple est absorbé sur toute la longueur de la jambière et en particulier sur le mollet. La section de la jambière étant une section tubulaire de grande dimension, son épaisseur peut être réduite et la composante tangentielle du couple de torsion est réduite.

D'autre part la luxation du genou, à la différence de la cassure ou fêlure des os de la jambe, peut résulter d'une chute avant, latérale ou en torsion. En conséquence, pour protéger le genou contre une luxation due à une chute avant, il suffit de bloquer dans le sens avant la rotation du fémur autour de l'articulation du genou, ce qui est réalisée avec la jambière présentant les caractéristiques cidessus. De plus, pour éviter la luxation du genou dans le cas d'une chute latérale ou en torsion, il faut emboîter le genou latéralement ainsi que la plus longue partie possible de la cuisse aboutissant au genou de manière qu'un couple de torsion tendant à s'exercer sur le genou, soit, notamment lorsque le genou a amorcé une flexion, absorbé par cet emboîtement et transmis par l'axe d'articulation de la jambière. En conséquence, et selon un autre mode de réalisation, la partie supérieure avant emboîtant le genou est prolongée à sa partie supérieure pour emboîter vers l'avant la partie de la cuisse aboutissant au genou et est articulée sur la demi-coquille avant autour d'un axe correspondant sensiblement à l'axe d'articulation du genou avec une butée limitant sa rotation vers l'avant.

De préférence, la jambière est constituée par deux demi-coquilles avant et arrière assemblées par des fermetures à crochet latérales. Selon une autre caractéristique, la demi-coquille avant est réglable en hauteur par rapport à la demi-coquille arrière. Pour obtenir ce réglage, l'une des demi-coquilles comporte un moyen d'accrochage constitué de plusieurs éléments étagés en hauteur avec un moyen d'accrochage complémentaire destiné à venir s'accrocher dans une des éléments dudit premier moyen d'accrochage fixé sur l'autre demi-coquille.

L'invention sera décrite plus en détail ci-après sous forme d'un mode de réalisation avec référence au dessin ci-annexé dans lequel:

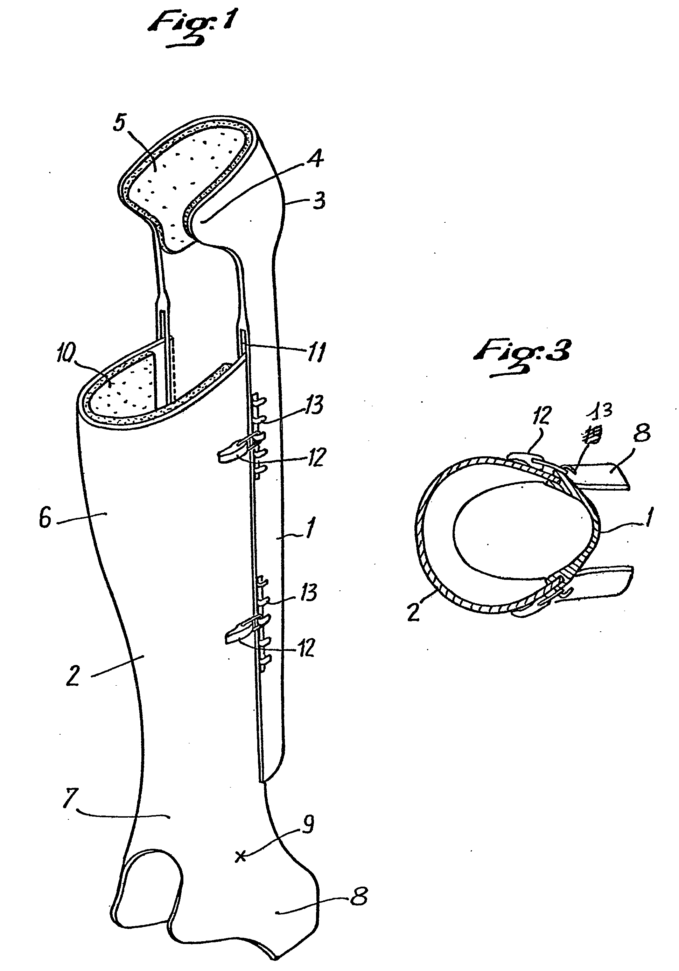

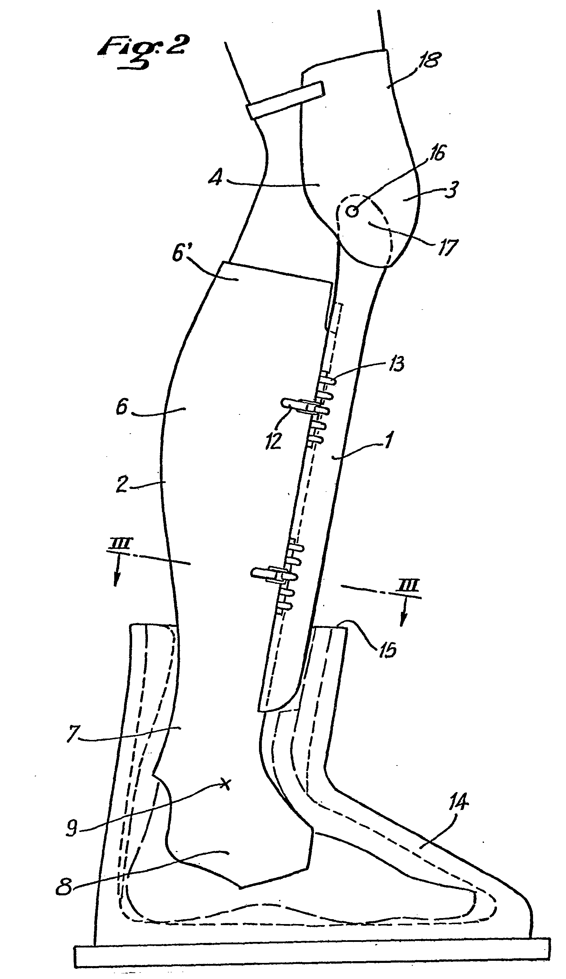

La figure 1 est une vue en perspective d'un premier mode de réalisation d'une jambière; la figure 2 est une vue en élévation latérale d'un autre mode de réalisation d'une jambière en position dans une chaussure; la figure 3 est une vue en coupe par III—III de la figure 2.

Dans le mode de réalisation représenté, à la figure 1, la jambière est constituée par une demi-coquille avant 1 et par une demi-coquille arrière 2.

La demi-coquille avant comporte une partie inférieure en forme de gouttière avec à sa partie supérieure une partie 3 emboîtant le genou et l'amorce de la partie inférieure avant de la cuisse par des oreilles latérales 4. La surface intérieure de la partie 3 et des oreilles latérales 4 est de préférence garnie avec un matelassage 5.

La demi-coquille arrière emboîte le mollet en 6 et la cheville en 7 et se prolonge par deux flasques latéraux 8 venant de part et d'autre du pied tout en laissant libre l'articulation autour de l'axe 9 des malléoles. Tout ou partie de la surface intérieure de la demi-coquille arrière peut être doublée par un matelassage 10.

Pour permettre une certaine adaptation de la jambière aux différentes longueurs de jambes, c'est-à-dire à la distance entre l'articulation des malléoles et le genou, les deux demi-coquilles peuvent être réunies par un emboîtement à tenon et languette 11 et solidarisées par des fixations rapides latérales comprenant, sur l'un des éléments, une attache à levier 12 et, sur l'autre élément, une pluralité de crochets étagés en hauteur 13 sur lesquels peut s'accrocher la boucle de l'attache.

Le mode de réalisation représenté à la figure 2 présente les mêmes éléments que ceux décrits avec référence à la figure 1 désignés par les mêmes références. Toutefois, la demi-coquille arrière 6 est prolongée en 6' pour monter jusqu'au bas du jarret et éviter une fracture de la partie supérieure du tibia dans une chute arrière et la genouillère 3 est articulée en deux points opposés 16 sur des oreilles 17 à la partie supérieure de la gouttière 1, ces deux articulations correspondant à un axe confondu avec l'axe de flexion du genou. La rotation vers l'avant de la pièce 3 autour de l'articulation 16 est limitée par butée de son bord inférieur sur la surface avant de la gouttière 1 et la genouillère 3 se prolonge vers le haut par une partie 18 enserrant sur une distance aussi grande que possible le devant et les faces latérales de la cuisse.

Comme cela ressort de la figure 2, dans le cas d'une chute avant, le poids du corps donne naissance à un couple dont le centre peut, en raison du blocage de la chaussure 14 sur le ski, être considéré comme ayant son axe sur l'axe 9 des malléoles. Sans la jambière, le couple est absorbé par une réaction s'exerçant au niveau du bord supérieur 15 de la tige de la chaussure, ce qui donne naissance aux fractures du tibia et du péroné au voisinage de ce niveau. Avec la jambière conforme à l'invention, la réaction se répartit sur toute la longueur de la jambe, sur le genou et éventuellement la partie de la cuisse voisine du genou. Il en est de même en cas de chute latérale. En cas de torsion, le couple est également absorbé, par suite de la réaction des flasques 8 qui ne peuvent tourner dans la tige, sur la totalité de la jambière, c'est-à-dire qu'il se traduit par une compression sur le mollet et sur les côtés de l'articulation du genou et de l'amorce du fémur.

**Revendications**

1. Une jambière pour la pratique du ski comportant une coquille formant jambière (1, 2) descendant dans le haut de la tige de la chaussure de ski, en dessous du niveau des malléoles, en s'emboîtant dans la chaussure par deux flasques latéraux (8) prenant appui contre les flancs de la tige, caractérisée en ce que, pour absorber au niveau de la cuisse les efforts latéraux et les couples de torsion, la coquille est constituée par deux demi-coquilles avant (1) et arrière (2) en un matériau rigide assemblées entre elles de façon rigide de manière à enserrer la jambe sur tout son pourtour, lesdits flasques latéraux (8) formant partie intégrante de la demi-coquille arrière (2) et la demi-coquille avant (1) étant solidaire d'une partie supérieure (3) avant emboîtant le genou en avant et latéralement.

2. Une jambière selon la revendication 1, caractérisée en ce que la partie supérieure (3) avant emboîtant le genou est prolongée à sa partie supérieure pour emboîter vers l'avant la partie (18) de la cuisse aboutissant au genou et est articulée sur la demi-coquille avant autour d'un axe (16) correspondant sensiblement à l'axe d'articulation du genou avec une butée limitant sa rotation vers l'avant.

3. Une jambière selon l'une quelconque des revendications 1 et 2, caractérisée en ce que les deux demi-coquilles avant et arrière sont assemblées par un emboîtement de leurs bords avec des fermetures à crochet (12, 13) s'opposant au déboîtement.

4. Une jambière selon l'une quelconque des revendications 1 à 3, charactérisée en ce que pour permettre le réglage en hauteur de la demi-coquille avant par rapport à la demi-coquille arrière, l'une des demi-coquilles 1 comporte un moyen d'accrochage (13) constitué de plusieurs éléments étagés en hauteur, un moyen d'accrochage (12) complémentaire destiné à venir s'accrocher dans un des éléments dudit premier moyen d'accrochage étant fixé sur l'autre demi-coquille (2).

## Patentansprüche

1. Beinschutz zum Skifahren mit einer den Beinschutz bildenden Schale (1, 2), die im oberen Bereich des Schaftes des Ski-Stiefels unterhalb der Höhe der Knöchel heruntergeht und sich in den Stiefel mit Hilfe von zwei seitlichen Flanschen (8) einpasst, die sich gegen die Flanken des Schaftes abstützen, dadurch gekennzeichnet, dass die Schale zur Aufnahme der seitlichen Kräfte und der Verdrehungsmomente in der Höhe des Oberschenkels aus zwei halben Schalen und zwar einer vorderen (1) und einer hinteren (2) aus einem steifen Material besteht, wobei diese halben Schalen untereinander steif verbunden sind, damit sie den gesamten Umfang des Beines einfangen, wobei die genannten seitlichen Flansche (8) einen integrierenden Bestandteil der hinteren halben Schale (2) bilden und die vordere halbe Schale (1) mit einem vorderen Oberteil (3) fest verbunden ist, der das Knie vorne und seitlich umgibt.

2. Beinschutz nach Anspruch 1, dadurch gekennzeichnet, dass der vordere Oberteil (3), der das Knie umgibt, nach oben verlängert ist, um nach vorne den Teil des Oberschenkels, der an das Knie angrenzt, zu umgeben und auf der vorderen halben Schale um eine Galenkachse (16) gelenkig angebracht ist, die ungefähr der Gelenkachse bes Knies entspricht, wobei ein Anschlag seine Drehbewegung nach vorne begrenzt.

3. Beinschutz nach Anspruch 1 oder 2, dadurch gekennzeichnet, dass beide halben Schalen vorne und hinten mit ihren Rändern mit Hilfe von Hakenverschlüssen (12, 13) ineinandergreifen, die einem Auseinandernehmen entgegenwirken.

4. Beinschutz nach Anspruch 1, 2 oder 3, dadurch gekennzeichnet, dass zur Höheneinstellung der vorderen halben Schale zur hinteren, die eine halbe Schale (1) ein Hakenmittel (13), das aus mehreren in der Höhe abgestuften Elementen besteht, sowie ein ergänzendes Hakenmittel (12) aufweist, das in eins der Elemente des genannten ersten Hakenmittels einhakt, das auf der anderen halben Schale (2) befestigt ist.

## Claims

1. A gaiter for skiing comprising a gaiterforming shell (1, 2) extending downwards into the top of the skiing boot upper, up to below the ankle level, by fitting into the boot through two side webs (8) bearing against the sides of the upper, characterized in that, so as to absorb at the thigh level the side forces and twisting moments, the shell consists of two front and rear half-shells (1, 2) of a rigid material assembled together in such rigid manner as to encompass the leg all around its periphery, said side webs (8) being integral with the rear half-shell (2) and the front half-shell (1) being integral with a front upper portion (3) encasing the knee at the front and on the sides.

2. A gaiter according to Claim 1, characterized in that said front upper portion (3) encasing the knee is extended at its upper portion to encase at the front that portion (18) of the thigh which terminates at the knee, and is pivotally mounted on said front half-shell about a pin (16) substantially corresponding to the joint axis of the knee, with an abutment acting to limit its forward rotation.

3. A gaiter according to either of Claims 1 and 2, characterized in that the two front and rear half-shells are assembled through interfitting of their edges, with hooked fixtures (12, 13) preventing dislocation.

4. A gaiter according to any of Claims 1—3, characterized in that for enabling vertical adjustment of said front half-shell with respect to the rear half-shell, one of said half-shells (1) comprises a hooking means (13) consisting of several vertically spaced elements, a mating hooking means (12) arranged for hooking up into one element of said first hooking means being secured onto the other half-shell (2).

Fig.1

Fig.3

*Fig. 2*